# EUROPEAN PATENT SPECIFICATION

(11) **EP 4 025 270 B1**
(45) Date of publication and mention of the grant of the patent: **01.11.2023**
(21) Application number: 20761289.6
(22) Date of filing: 31.08.2020
(51) Int. Cl.: A61M 1/06

(54) **ELECTRIC BREAST PUMP**
ELEKTRISCHE BRUSTPUMPE
TIRE-LAIT ÉLECTRIQUE

(30) Priority: 03.09.2019 EP 19195099
(43) Date of publication of application: 13.07.2022
(73) Proprietor: Koninklijke Philips N.V., 5656 AG Eindhoven (NL)
(72) Inventor: HESEN, Robertus, Gerardus, Johannes, Antonius, 5656 AE Eindhoven (NL)
(74) Representative: Philips Intellectual Property & Standards
(86) International application number: PCT/EP2020/074198
(87) International publication number: WO 2021/043710

(56) References cited:
- US-A1- 2016 339 156
- US-A1- 2018 104 395

## Description

### FIELD OF THE INVENTION

The present invention relates to an electric breast pump and a computer program for operating an electric breast pump.

### BACKGROUND OF THE INVENTION

Electric breast pumps are used by women in order to express milk from their breast to feed the milk to their babies at a desired time. A breast pump typically comprises an expression kit with a funnel into which the breast of the user is placed. The expression kit also comprises a bottle or vessel into which the expressed milk can flow. The expression kit is coupled to a vacuum source to enable the expression of milk. The vacuum source provides a vacuum which is applied to the breast in the funnel to extract milk. The breast pump may comprise two operating modes, namely a stimulation mode and an extracting mode. During the stimulation mode, the milk ejection reflex is stimulated. When the breast pump is activated, a vacuum source like a vacuum pump is activated and generates a vacuum. The vacuum pump is typically activated by an electrical motor. During the extracting mode, a pumping cycle of the vacuum pump comprises a pumping period followed by an aerate period. During the pumping period, the vacuum pump is activated while it is not activated during the aerate period. As a user of the breast pump is typically immobile during the extraction of milk, a reduced time for extracting the milk would be appreciated. Controlling the exact vacuum level required for the stimulation mode and the extraction mode is difficult as it can be difficult to determine the exact vacuum level.

US 2004/127845 A1 discloses a breast pump system with an electrical motor which speed can be controlled. The speed of the motor can be programmed within available ranges. Further relevant prior art is disclosed in documents US2018/104395 A1 and US2016/339156 A1.

### SUMMARY OF THE INVENTION

It is an object of the invention to provide an electric breast pump which enables a fast and efficient milk extraction. It is further an aspect of the invention to provide an electric breast pump with an increased battery life.

An electrical breast pump according to the present invention comprises the technical features as defined in independent claim 1. A computer program according to the present invention comprises the technical features as defined in independent claim 6. The breast pump comprises a vacuum source with a vacuum pump operated by an electrical motor and an aerate valve. Furthermore, a controller is provided which is adapted to control the vacuum pump and the aerate valve e.g. in a stimulation mode and an extracting mode. A drive circuit serves to supply a motor supply voltage to the electrical motor to operate the electrical motor of the vacuum pump e.g. in a stimulation mode and an extracting mode for milk extraction. A pumping cycle can comprise a pumping period and an aerate period. When the motor supply voltage is switched off during the pumping cycle, the drive circuit serves to detect an electromotive force induced voltage at the electrical motor. The detected induced voltage can be used by the controller to adapt the control of the electrical motor and/or of the aerate valve.

With such a breast pump it is possible to increase the battery life of a battery driven electrical breast pump.

An electromotive force (EMF) induced voltage is generated when the input voltage is switched off (reduced to zero) but the rotor of the motor is still turning. This voltage can be used e.g. to determine a previous motor speed. Furthermore, the determination of the electromotive force can be used to decide when to activate the motor again. When some electromotive force induced voltage is present, an improved ramping up or starting of the motor can be reduced or achieved. In particular, a high inrush current at the start of the motor can be avoided. Furthermore, by avoiding a high current, the required energy can be reduced thus increasing the battery life of a battery driven electrical breast pump.

After the motor of the vacuum source has stopped (the applied voltage is reduced to zero), the electromotive force induced voltage is determined. Based on this voltage, the speed of the motor at which it has run can be derived. The higher the induced voltage, the higher the previous speed of the motor.

According to an embodiment of the invention, the controller is configured to reactivate the vacuum pump by supplying the motor supply voltage to the electrical motor when the drive circuit is still detecting the electromotive force induced voltage. This is performed in order to keep the pumping motor (slowly) running during the aerate period. This is advantageous as the start up of the pump in a subsequent pumping period is quicker. In particular, the ramp up of the electrical motor rotation speed is faster. This will lead to an increase in the vacuum value and can shorten the pumping period. It should be noted that it is much easier and faster to start up the electrical pump from a slowly rotating mode than from a complete standstill. This is in particular advantageous as it can avoid a high inrush current at the starting point if the motor is at a complete standstill. Furthermore, a high current peak at the start of the pumping circuit can be avoided leading to an increased battery life.

According to an embodiment, the controller is configured to determine a speed of the electrical motor at which the motor has previously run (just before the stoppage of the motor) based on the detected induced voltage. Based on the detected induced voltage of the electro motive force EMF after the electrical motor has switched off, a duration that the electrical motor has run in the previous pumping period can be determined such that a vacuum must have been generated in the pump system, the vacuum pump and extraction kit. This applies as long as air hoses to the extraction kit are not broken and are properly connected. The electrical motor is reactivated again during a current pumping cycle e.g. based on the determined previous speed of the electrical motor.

With the electric breast pump according to an embodiment, a cost effective breast pump can be provided which allows an improved determination of the applied vacuum levels. The rotation speed of the electrical motor can be measured or determined based on the detected electromotive force (EMF) induced voltage. The controller can keep the electrical motor of the vacuum pump slightly running in the Aerate period so that during start up in the beginning of the pump period, the pump is started up faster and can reach the desired vacuum level sooner.

A drive unit or drive circuit is provided for applying a motor supply voltage to input terminals of the electrical motor. The drive circuit is capable of supplying the required motor supply voltage to the motor according to the required voltage profile during the start up and off time of the motor. In addition, the drive circuit is able to measure or detect the electromotive force induced voltage at input terminals of the motor when the motor supply voltage is switched off or is reduced to zero. The drive circuit can under control of the controller supply the required voltage profile to the motor during a pumping cycle where the motor of the vacuum pump is switched on and switched off in a periodic fashion. The drive circuit can also be integrated into the controller. Hence, the controller can provide the supply voltage and detect the electromotive force induced voltage at input terminals of the motor when the motor supply voltage is switched off or is reduced to zero.

According to an embodiment, the electric breast pump comprises a power supply for example in form of a rechargeable battery or a battery. This is advantageous as such a breast pump is mobile and does not need to be connected to the mains to enable an operation. Accordingly, such that a breast pump would be more flexible than a standard breast pump.

It should be that noted the motor for the breast pump must be started for each pump period and stopped again after the pump period. Accordingly, the pump will be activated and deactivated a great number of times.

A computer program for operating a breast pump is also provided. The computer program comprising program code means for causing an electrical breast pump to carry out the steps of the method of operating a breast pump as defined above, when the computer program is run on a computer controlling the breast pump.

The breast pump according to an embodiment is advantageous as it does not require additional pressure sources or the measuring of the input voltage of the motor to determine an exact vacuum level.

According to the present disclosure a controller for an electrical breast pump having a vacuum source comprising a vacuum pump with an electrical motor and an aerate valve is provided. The controller is configured to control the operation of the vacuum pump based on a detected electromotive force induced voltage at the electrical motor, when a motor supply voltage is switched off.

Other aspects of the invention are described in the dependent claims. Further advantages and embodiments of the invention will now be elucidated with reference to the drawings.

These and other aspects will be apparent from and elucidated with reference to the embodiments described hereinafter.

### BRIEF DESCRIPTION OF THE DRAWINGS

In the following drawings:
Fig. 1 shows a block diagram of a breast pump according to an embodiment,
Fig. 2 shows a graph depicting a voltage profile for a breast pump,
Fig. 3 shows a graph depicting a voltage and current profile of a breast pump in a pumping cycle.
Fig. 4 shows a graph depicting an enlarged portion of the voltage and current profile of Fig. 3 in a pumping period, and
Fig. 5 shows a graph depicting another enlarged portion of the voltage and current profile of Fig. 3 in an aerate period.

### DETAILED DESCRIPTION OF EMBODIMENTS

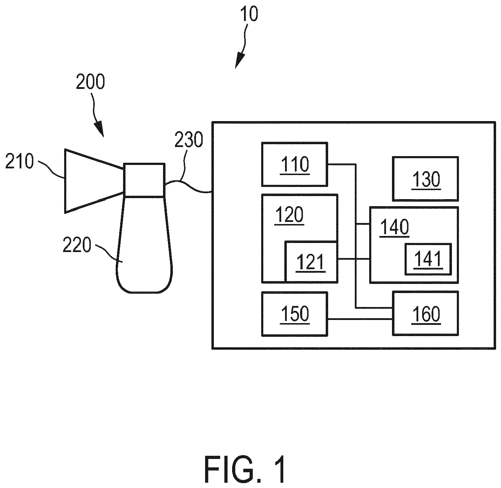

Fig. 1 shows a block diagram of a breast pump according to an embodiment. The breast pump 10 comprises an expression kit 200 and a vacuum source 100. The expression kit 200 comprises at least one funnel 210 for receiving a breast of a user, and a bottle 220 for collecting expressed milk. The expression kit 200 is coupled to the vacuum source 100 via a vacuum conduit 230.

The vacuum source 100 comprises a controller 110, a vacuum pump 120 with an electrical motor 121. The electrical motor 121 can be driven by a drive unit 140. Furthermore, the vacuum source 100 comprises a power supply 130 and an aerate valve 150. The aerate valve 150 can be driven by the valve drive unit 160 that is also controlled by the controller 110.

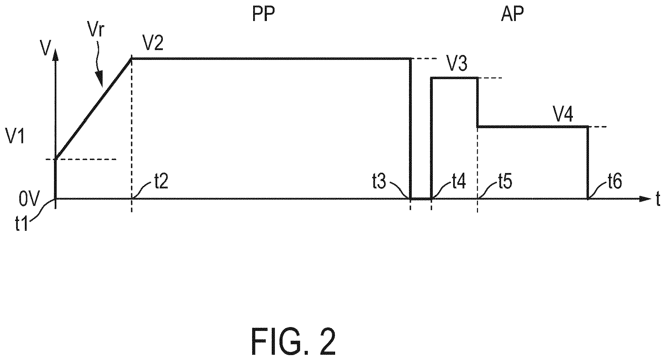

Fig. 2 shows a graph depicting a voltage and current profile for a breast pump according to Fig. 1. During operation, the controller 110 controls the operation of the vacuum pump 120 via the drive circuit 140. and the aerate valve 150 via the valve drive circuit 160. In particular, the breast pump 10 has a stimulation mode and an extraction mode for stimulating the milk ejection reflex. When the breast of a user is placed into the funnel 210 and when the vacuum source 100 is activated, the vacuum pump 120 creates a vacuum which is supplied via the vacuum conduit 230 to the breast placed in the funnel 210.

The drive circuit 140 serves to provide a voltage profile of a motor supply voltage for the electrical motor 121 of the vacuum pump 120. When the vacuum pump is to be started, the drive circuit 140 provides a voltage ramp to the electrical motor 121. Once the voltage has reached its desired value, the voltage is kept constant until the pumping period has ended and the motor supply voltage is switched off. When the motor supply voltage has been switched off, an electromotive force induced voltage can be measured by the drive circuit 140 at the input terminals of the electrical motor 121. Thus, the drive circuit 140 can have two operating modes, namely a first operating mode for supplying the required voltage profile to the electrical motor and a second operating mode where the motor supply voltage is switched off and the drive circuit can detect an electromotive force induced voltage at the input terminal of the motor 121.

The measured electromotive force (EMF) induced voltage can be used to determine the motor speed of the electrical motor 121 before the motor supply voltage Vp was switched off. In other words, the measured induced voltage can be used to retrospectively determine the motor speed of the rotor. This information can be used for example by the controller to determine or derive the voltage level at the funnel of the expression kit 200. If the determined voltage level does not correspond to the required voltage level, then the controller 110 can initiate a change of the motor supply voltage as provided by the drive circuit 140.

The drive circuit 140 can be a dedicated unit or can be part of the controller 110.

In addition or alternatively, the measured induced voltage can be used by the controller 110 to decide when to reactivate the electrical motor 121 for the next pumping period. It is in particular advantageous to reactivate the pumping period for the vacuum pump by supplying the required voltage profile of the motor supply voltage to the motor by means of the drive circuit 140 when the induced voltage is still present. That is performed during the aerate period. This is advantageous as applying the low required voltage profile to the electrical motor when the induced voltage is above zero will decrease the initial value of the current peak flowing through the motor at start up of the next pumping period. This is advantageous as applying a low required voltage profile to the electrical motor when the induced voltage is above zero increases the vacuum level in the next pumping period. In addition, the time required for the next pumping period can be reduced.

After the stimulation mode, the extraction mode is activated. In the extraction mode, a pumping cycle is continuously repeated. During each pumping cycle, a pumping period PP and an aerate period AP are present. During the pumping period PP, the controller 110 controls the drive unit 140 to supply a motor supply voltage for the motor 121 of the vacuum pump 120. During a first stage at the point of time t1, an initial voltage value V1 is set or applied to the motor 121 of the vacuum pump 120. The voltage will then increase from V1 to V2 with a voltage ramp Vramp until t2. At t2, the voltage reaches its nominal value V2 and is substantially constant until t3, when the voltage is reduced to zero.

During the aerate period AP and at time t4, a voltage of the valve is at a fixed level V3 so that the valve is opens. At time t5, the voltage is reduced to V4 and is reduced to zero at t6.

The electromotive force induced voltage of a still running electrical motor 121 is determined when the motor supply voltage is switched off. The induced voltage can occur within a certain time period. The amount of the induced voltage can be used to determine the motor speed of the electrical motor before the motor supply voltage was switched off. The drive circuit 140 can comprise a detection circuit 141 with an e.g. 1/2 voltage resistor divider for a matching the range of the analogue to digital converter ADC of a (micro-)controller of 3.0V. Furthermore, the detection circuit 141 can comprise a filter for filtering out the pulse width modulation PWM switching frequency or spikes of the motor. Moreover, the analog digital converter ADC input can be connected to an input of the microcontroller.

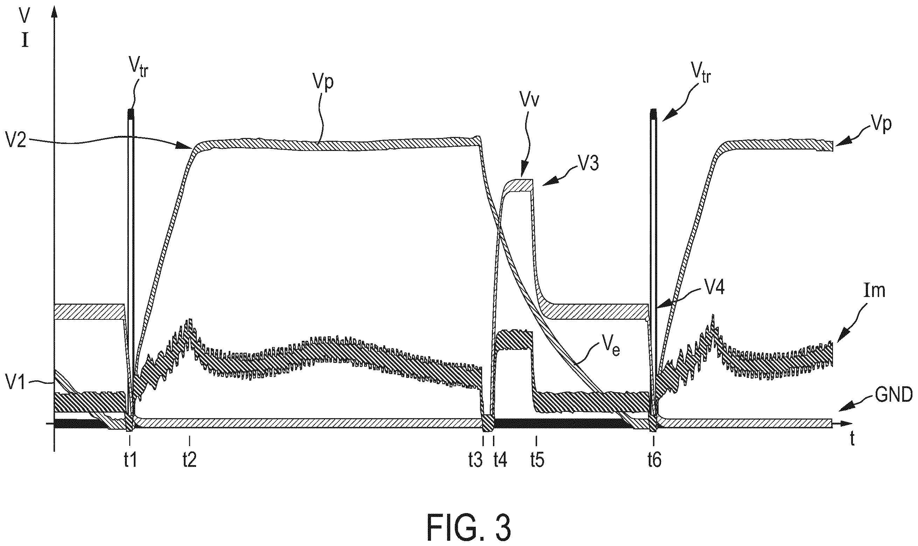

Fig. 3 shows a graph depicting a voltage and current profile of a breast pump. In Fig. 3, the trigger Voltage pulse Vtr for the Osciloscope is at the begin of every pumping cycle and not relevant for the operation of the breast pump but only for triggering the oscilloscope. In Fig. 3, the motor supply voltage Vp, the voltage of the valve Vv and a motor current Im is depicted. The valve Voltage Vv is set to V3 when the valve is open and set to V4 when the valve is kept open. Furthermore, the electromotive force induced voltage Ve in the aereta period is also depicted. In particular, the electromotive force induced voltage Ve can be measured from t3 to the end time t6 (or till t1 of the next pumping cycle), i.e. when the motor supply voltage Vp is reduced to zero. As can be seen in Fig. 3, between t1 and t2, the voltage is raised from V1 to V2 and then between t2 and t3, the voltage is kept substantially constant. At t3, the supply voltage is reduced to zero and the electromotive force induced voltage Ve can be measured. Between t4 and t5, the valve is activated by supplying the valve open voltage V3. At t5, the valve voltage is reduced to V4. The valve voltage is set to zero at time t6.

In Fig. 3 also the current Im is depicted. Between t1 and t2, the current is increased with a peak at t2. Thereafter, the current Im reduces and at t3, the current is reduced to zero. The current Im is the current through the pump motor drive control 140.

In Fig. 3, the voltage profile is depicted. In particular, the ramp up voltage starts from V1 and ends at V2. V2 then corresponds to the pump final voltage. However, when the voltage has reached V2 (the motor is running at maximum speed), the current will drop. When the motor supply voltage is switched off and the electromotive force induced voltage is induced at the input terminals of the motor, the current drops. Furthermore, a higher current is present when air needs to be pumped out of the chambers and thus the load of the electrical motor 121 is higher. At the end of the period, the current will drop as the pump only has to pump vacuum at a reduced pressure and the flow is almost zero.

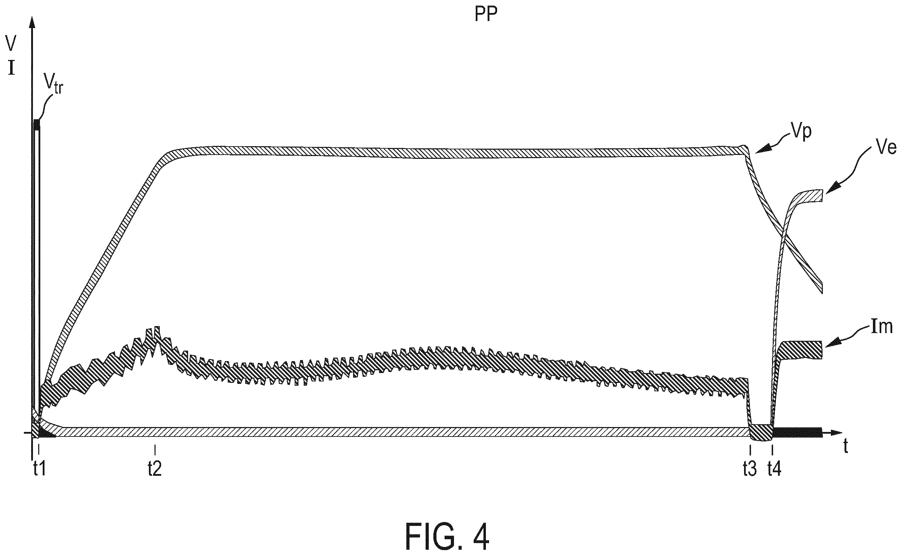

Fig. 4 shows a graph depicting an enlarged portion of the voltage and current profile of Fig. 3. In Fig. 4, in particular a pump period is depicted. The pump period PP stretches from t1 to t3. Accordingly, the motor supply voltage Vp is increased with a ramp between t1 and t2. At the same time, the current Im increased with a peak at approximately t2. At t3, the motor supply voltage is switched off and the electromotive force induced voltage Ve can be measured.

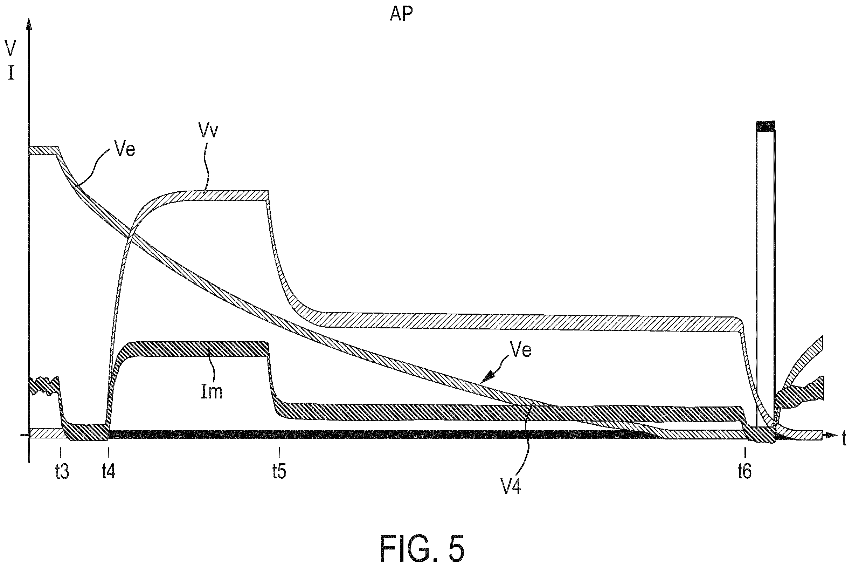

Fig. 5 shows a graph depicting another enlarged portion of the voltage and current profile of Fig. 3. In Fig. 5, the aerate period AP is depicted. Accordingly, the time between t4 and t6 is depicted. From t3 onwards, the electromotive force induced voltage Ve is present. Furthermore, between t4 and t5, the voltage of the valve Vv is set to V3. Between t5 and t6, the voltage of the valve is decreased to V4.

After the input voltage of the motor has been switched off or reduced to zero, a voltage present at the motor is detected or measured. This voltage corresponds to a voltage induced by the electromotive forces of the motor. As the rotor of the motor is still running even after the input voltage has been switched off, the still turning rotor induces a voltage at the stator of the motor. This voltage can be detected at the input terminals of the motor. The detected induced voltage can be used to retrospectively measure the previous speed of the motor. Furthermore, the induced voltage can be used to activate the motor again at a low voltage level at the next instance of the periodic pumping cycle. Here it is in particular advantageous to activate the motor again when there is still some electromotive force induced voltage present. This can help to reduce a high inrush current at the start up of the motor at the next pumping period. By reducing the high inrush current, battery power may be saved.

Furthermore, the induced voltage can be measured and used to activate the motor at a low voltage level in the last part of the aerate period. Here it is in particular advantageous to activate the motor again when there is still some electromotive force induced voltage present. This helps to get the pump run up quicker and so getting a lower vacuum level or reaching the desired vacuum level earlier. The latter will have a shorter pumping period.

Furthermore, the induced voltage can be measured. When measured directly after t3 and at some more small fixed times after t3, a speed rotating profile can be derived. Together with the knowledge that the pump had run for a certain time (time t3 - t2 (ort1)), one can conclude that the pump has pumped vacuum in the hose line and extraction kit. So that there had to be vacuum in the vacuum part of the system. Of course only if the external vacuum hose from the vacuum unit 100 to the extraction kit 200 was not broken or not properly connected.

According to an embodiment a controller for an electrical breast pump having a vacuum source (100) comprising a vacuum pump (120) with an electrical motor (121) and an aerate valve (150) is provided. The controller is configured to control the operation of the vacuum pump (120) based on a detected electromotive force induced voltage at the electrical motor (121), when a motor supply voltage is switched off.

In the claims, the word "comprising" does not exclude other elements or steps, and the indefinite article "a" or "an" does not exclude a plurality.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. Electrical breast pump (10), comprising
a vacuum source (100) having a vacuum pump (120) with an electrical motor (121) and an aerate valve (150), and
a controller (110) configured to control an operation of the vacuum pump (120) and the aerate valve (150,
wherein a pumping cycle comprises a pumping period (PP) of the vacuum pump (120) and an aerate period (AP) during which the aerate valve (150) is switched on and the vacuum pump (120) is inactive and the electrical motor (121) is switched off,
a drive circuit (140) which is configured to supply a motor supply voltage for the electrical motor (121) under the control of the controller (110),
wherein the drive circuit (140) is also configured to detect an electromotive force induced voltage at the electrical motor (121), when the motor supply voltage is switched off,
wherein the controller (110) is configured to adapt the control of the operation of the vacuum pump (120) and/or the aerate valve (150) based on the detected induced voltage.

2. Electrical breast pump (10) according to claim 1, wherein
the controller (110) is configured to reactivate the vacuum pump (120) by supplying the motor supply voltage when the drive circuit (140) is still detecting the electromotive force induced voltage.

3. Electrical breast pump (10) according to claim 1 or 2, wherein
the controller (110) is configured to determine a speed of the electrical motor (121) at which the electrical motor (121) has run in a previous pumping cycle based on the detected induced voltage and to reactivate the electrical motor (121) again during a current pumping cycle based on the determined previous speed of the electrical motor (121).

4. Electrical breast pump (10) according to any one of the claims 1 to 3, further comprising
an expression kit (200) having at least one funnel (210) adapted for receiving a breast of a user, a bottle (220) adapted to collect extracted milk and a port for a vacuum conduit (230) through which the expression kit is coupled to the vacuum source (100).

5. Electrical breast pump (10) according to any one of the claims 1 to 4, further comprising
a power supply (130) in form of a rechargeable battery or a battery.

6. A computer program for operating a breast pump, the computer program comprising program code means for causing an electrical breast pump as defined in claim 1 to carry out the following steps of a method of operating a breast pump, when the computer program is run on a computer controlling the breast pump:
controlling an operation of the vacuum pump (120) in a stimulation mode and an extraction mode for milk extraction,
wherein a pumping cycle comprises a pumping period (PP) and an aerate period (AP),
supplying a motor supply voltage for the electrical motor (121) of the vacuum pump (120) by a drive circuit (140),
detecting an electromotive force induced voltage after the motor supply voltage is switched off, and
controlling the operation of the vacuum pump (120) based on the detected induced voltage.

## Patentansprüche

1. Elektrische Brustpumpe (10), umfassend
eine Vakuumquelle (100) mit einer Vakuumpumpe (120) mit einem Elektromotor (121) und einem Belüftungsventil (150), und
eine Steuerung (110), die konfiguriert ist, um den Betrieb der Vakuumpumpe (120) und des Belüftungsventils (150) zu steuern,
wobei ein Pumpzyklus eine Pumpperiode (PP) der Vakuumpumpe (120) und eine Belüftungsperiode (AP) umfasst, während der das Belüftungsventil (150) eingeschaltet und die Vakuumpumpe (120) inaktiv ist und der Elektromotor (121) ausgeschaltet ist,
eine Antriebsschaltung (140), die konfiguriert ist, um unter Steuerung der Steuerung (110) eine Motorversorgungsspannung für den Elektromotor (121) bereitzustellen,
wobei die Antriebsschaltung (140) ferner konfiguriert ist, um eine durch eine elektromotorische Kraft induzierte Spannung am Elektromotor (121) zu erfassen, wenn die Motorversorgungsspannung ausgeschaltet ist,
wobei die Steuerung (110) konfiguriert ist, um die Steuerung des Betriebs der Vakuumpumpe (120) und/oder des Belüftungsventils (150) basierend auf der erfassten induzierten Spannung anzupassen.

2. Elektrische Brustpumpe (10) nach Anspruch 1, wobei
die Steuerung (110) konfiguriert ist, um die Vakuumpumpe (120) durch Zuführen der Motorversorgungsspannung zu reaktivieren, wenn die Antriebsschaltung (140) immer noch die durch die elektromotorische Kraft induzierte Spannung erfasst.

3. Elektrische Brustpumpe (10) nach Anspruch 1 oder 2, wobei
die Steuerung (110) konfiguriert ist, um anhand der erfassten induzierten Spannung eine Drehzahl des Elektromotors (121) zu ermitteln, mit der der Elektromotor (121) in einem vorherigen Pumpzyklus gelaufen ist, und den Elektromotor (121) während eines aktuellen Pumpzyklus basierend auf der ermittelten vorherigen Drehzahl des Elektromotors (121) zu reaktivieren.

4. Elektrische Brustpumpe (10) nach einem der Ansprüche 1 bis 3, weiterhin umfassend:
ein Expressionskit (200) mit mindestens einem Trichter (210) zur Aufnahme einer Brust einer Benutzerin, einer Flasche (220) zum Auffangen abgesaugter Milch und einem Anschluss für eine Vakuumleitung (230), über die das Expressionskit mit der Vakuumquelle (100) verbunden ist.

5. Elektrische Brustpumpe (10) nach einem der Ansprüche 1 bis 4, weiterhin umfassend:
eine Stromversorgung (130) in Form eines Akkus oder einer Batterie.

6. Computerprogramm zum Betreiben einer Brustpumpe, wobei das Computerprogramm Programmcodemittel umfasst, um zu veranlassen, dass eine elektrische Brustpumpe nach Anspruch 1 die folgenden Schritte eines Verfahrens zum Betreiben einer Brustpumpe ausführt, wenn das Computerprogramm auf einem Computer ausgeführt wird, der die Brustpumpe steuert:
Steuern eines Betriebs der Vakuumpumpe (120) in einem Stimulationsmodus und einem Extraktionsmodus zur Milchextraktion,
wobei ein Pumpzyklus eine Pumpperiode (PP) und eine Belüftungsperiode (AP) umfasst,
Bereitstellen einer Motorversorgungsspannung für den Elektromotor (121) der Vakuumpumpe (120) durch eine Antriebsschaltung (140),
Erfassen einer durch eine elektromotorische Kraft induzierten Spannung, nachdem die Motorversorgungsspannung ausgeschaltet wurde, und
Steuern des Betriebs der Vakuumpumpe (120) basierend auf der erfassten induzierten Spannung.

## Revendications

1. Tire-lait électrique (10), comprenant
une source de vide (100) comportant une pompe à vide (120) avec un moteur électrique (121) et une vanne d'aération (150), et
un dispositif de commande (110) configuré pour commander un fonctionnement de la pompe à vide (120) et de la vanne d'aération (150),
où un cycle de pompage comprend une période de pompage (PP) de la pompe à vide (120) et une période d'aération (AP) pendant laquelle la vanne d'aération (150) est activée et la pompe à vide (120) est inactive et le moteur électrique (121) est arrêté,
où un circuit d'entraînement (140) est configuré pour fournir une tension d'alimentation de moteur pour le moteur électrique (121) sous le commande du dispositif de commande (110),
où le circuit d'entraînement (140) est également configuré pour détecter une tension électrique induite par une force électromotrice au niveau du moteur électrique (121), lorsque la tension d'alimentation du moteur est coupée,
où le dispositif de commande (110) est configuré pour adapter la commande du fonctionnement de la pompe à vide (120) et/ou de la vanne d'aération (150) sur la base de la tension électrique induite détectée.

2. Tire-lait électrique (10) selon la revendication 1, dans lequel
le dispositif de commande (110) est configuré pour réactiver la pompe à vide (120) en fournissant la tension d'alimentation du moteur lorsque le circuit d'entraînement (140) détecte encore la tension électrique induite par la force électromotrice.

3. Tire-lait électrique (10) selon la revendication 1 ou 2, dans lequel
le dispositif de commande (110) est configuré pour déterminer une vitesse du moteur électrique (121) à laquelle le moteur électrique (121) a fonctionné dans un cycle de pompage précédent sur la base de la tension électrique induite détectée et pour réactiver le moteur électrique (121) à nouveau pendant un cycle de pompage en cours sur la base de la vitesse précédente déterminée du moteur électrique (121).

4. Tire-lait électrique (10) selon l'une quelconque des revendications 1 à 3, comprenant en outre
un kit d'expression (200) comportant au moins un entonnoir (210) adapté pour recevoir le sein d'une utilisatrice, un biberon (220) adapté pour collecter le lait extrait et un orifice pour un conduit sous vide (230) à travers lequel le kit d'expression est couplé à la source de vide (100).

5. Tire-lait électrique (10) selon l'une quelconque des revendications 1 à 4, comprenant en outre
une alimentation électrique (130) sous la forme d'une batterie rechargeable ou d'une batterie.

6. Programme informatique pour faire fonctionner un tire-lait, le programme informatique comprenant des moyens de code de programme pour amener un tire-lait électrique tel que défini dans la revendication 1 à exécuter les étapes suivantes d'un procédé de fonctionnement d'un tire-lait, lorsque le programme informatique est exécuté sur un ordinateur commandant le tire-lait:
commander un fonctionnement de la pompe à vide (120) dans un mode de stimulation et un mode d'extraction pour l'extraction du lait,
où un cycle de pompage comprend une période de pompage (PP) et une période d'aération (AP),
fournir une tension d'alimentation au moteur électrique (121) de la pompe à vide (120) par un circuit d'entraînement (140),
détecter une tension électrique induite par une force électromotrice après la coupure de la tension d'alimentation du moteur, et
commander le fonctionnement de la pompe à vide (120) sur la base de la tension électrique induite détectée.
